# EUROPEAN PATENT SPECIFICATION

(11) **EP 2 296 612 B1**
(45) Date of publication and mention of the grant of the patent: **11.10.2017**
(21) Application number: 09794702.2
(22) Date of filing: 21.01.2009
(51) Int. Cl.: A61K 8/19, A61K 8/25, A61Q 11/00, A61P 1/02, A61K 6/00, A61K 33/06, A61K 33/14

(54) **THERAPEUTIC AND PROPHYLACTIC FORMULATION FOR ORAL CARE**
THERAPEUTISCHE UND PROPHYLAKTISCHE MUNDPFLEGEFORMULIERUNG
FORMULATION THÉRAPEUTIQUE ET PROPHYLACTIQUE POUR SOIN BUCCAL

(30) Priority: 07.07.2008 EA 200801824
(43) Date of publication of application: 23.03.2011
(73) Proprietor: Obthestvo S Ogranichennoyj Otvetstvennostjyu "WDS", Moscow 123592 (RU)
(72) Inventor: MANASHEROV, Tamaz Omarovich, Moskva 119296 (RU); MATELO, Svetlana Konstantinovna, Moskovskaya obl. 143422 (RU); KUNIN, Anatoliyj Abramovich, Voronezh 394000 (RU); GROSSER, Aleksandr Vladimirovich, Moskovskaya obl. 143300 (RU)
(74) Representative: Windsor, Louise
(86) International application number: PCT/RU2009/000012
(87) International publication number: WO 2010/005338

(56) References cited:
- WO-A1-02/30381
- WO-A1-99/33437
- WO-A1-2007/061328
- EA-A1- 200 701 152
- GB-A- 136 442
- KR-A- 20040 041 471
- KELLY EDENBRIDGE M P ET AL: "The effect of remineralizing solutions on tooth wear in vitro", JOURNAL OF DENTISTRY, ELSEVIER, AMSTERDAM, NL, vol. 16, no. 3, 1 June 1988 (1988-06-01), pages 147-149, XP026276392, ISSN: 0300-5712, DOI: 10.1016/0300-5712(88)90011-5 [retrieved on 1988-06-01]

## Description

### Field of application

The invention relates to stomatology in particular to therapeutic and prophylactic formulations for oral cavity care.

### Background of invention

There is known formulation in the form of rinse, tooth paste or gel for prophylaxis of solid teeth tissues diseases, caries in particular. The formulation includes polyatomic alcohols, xylitol and sodium fluoride specifically. (US 6238648 B1, IPC 7 A61K8/34, 2001).

There is known formulation in the form of gel which is recommended for remineralization of solid teeth tissues containing xylitol, calcium glycerophosphate as a source of calcium and phosphorus and a microelement named magnesium acting as activator phosphatase. (RU 2311168 C2, IPC 8 A61K 8/97, 2007).

WO 99/ 33437 A1 discloses a toothpaste tablet comprising from about 20 to about 80 % of a polishing agent, from about 0.2 to about 5.5 % of a thickening agent, and from about 30 to about 80 % of a tableting carrier. The toothpaste tablet composition according to example II comprises magnesium stearate (2.5 wt%), sodium alkyl sulphate and sodium saccharine (1.0 and 0.13 wt%, respectively), synthetic silicate (4.6 wt%) and potassium citrate (1.0 wt%).

Known formulations, as well as formulations containing fluorides, do not provide for high-level mineralization of solid teeth tissues and include xylitol rather expensive now.

### Substance of invention

Technical result of invention lies in practical realization of high-efficient remineralizing formulation using available and cheap components. Due to absence of fluorides it can be recommended to patients suffering from fluorosis, endocrine system diseases for the purpose of caries prophylaxis and non-carious affections.

The technical result specified can be achieved by therapeutic and prophylactic formulation for oral care which contains acceptable active and inert components. To the group of active components there belongs complex including sources of elements of magnesium, sodium, silica and potassium in quantity 1-11 wt. %. Here are ratios of atomic masses of elements of magnesium, sodium, silica, potassium, parts of active components complex, correspondingly, 21.3:9.3:1.67:1.

As a source of magnesium element there is used magnesium chloride or magnesium glycerophosphate or magnesium citrate. As a source of sodium element there is used sodium chloride or sodium silicate. As a source of silica element there is used sodium metasilicate or potassium metasilicate. As a source of potassium element there is used potassium silicate.

Having magnesium, silica, sodium or potassium in a certain ratio in its formula tooth pastes and gels make it possible to normalize metabolic processes in solid teeth tissues. High content of magnesium increases progressively level of such elements as calcium and phosphorus relevant for solid tissues. Appliance of formulations with the complex mentioned contributes to formation of physiological, transparent, thin film on teeth surface with high mineralizing properties which has no cariogenic activity.

It is common knowledge that fluorine-containing means (tooth pastes, gels, varnishes) is effective for caries prophylaxis at a young age for 20% of population, for the rest 80% it is not effective. Fluoride appliance can result adverse for people of 40-45 years and upwards. Furthermore it may be dangerous to apply fluorine-containing tooth pastes and other means in regions with drinking water containing much fluorine. While studying fluorine content in drinking water on the territory of the Check Republic researchers have found out that content of this element is not optimal (0.1-0.4 mg/l). However carious affections ranged significantly, which depended on magnesium content in drinking water. It has been suggested that magnesium should perform the function of structural substrate as well as enzymatic processes activator carrying out mineralization of solid teeth issues. (Vejrosta Z., Sindelka Z., Feiler M., Vilser M. Sledovani karivosti zubu u deti pijichich vodu s vysokym obsahem borciku. Cs. Stomat., 1975, 95, Nº5, 346-354. Reference to N.A. Kodola, Microelements in Teeth Caries Prophylaxis, Kiev, "Zdorovie", p. 98,1979). Magnesium is directly linked to biological mineralization of bones and teeth. It has been demonstrated that magnesium has direct influence on biomineralization, modifying crystal size and orientation. (Hans-Peter Wiesmann et al. Magnesium in Newly Formed Dentin Mineral of Rat Incisor. Journal of bone and Mineral Research, Vol. 12, 3, 1997).

Silica initiates mineralization processes especially at early stages. Even at low calcium content silica accelerates its assimilation processes. Polysilicic acids solutions and even silica large particles cause spontaneous calcium and phosphate precipitation in the form of hydroxyapatite (J.J.M. Damen, J.M. Ten Cate The Effect of Silicic Acid on Calcium Phosphate Precipitation. J. Dent. Res. 68(9):1355-1359, September, 1989).

At best formulation active components content is 2-9 wt. %.

Formulation can be prepared in the form of a tooth paste, gel or liquid.

Optimum realization of invention supposes preparation of therapeutic and prophylactic formulation for oral care in the form of gel containing inert components such as, wt. %:
Moisturizing component - 5 - 70,
Gel-forming component - 0.5 - 3.0,
Surface-active component - 0.5-3.0,
Flavor filler - 0.05 - 0.3,
Preservative - 0.01 - 0.5.

If formulation were a paste it contains following inert components, wt %:
Abrasive component - 10 - 30,
Moisturizing component - 5 - 70,
Gel-forming component - 0.5 - 3.0,
Surface-active component - 0.5 - 3.0,
Flavor filler - 0.5 - 2.

If formulation were liquid it comprises following inert components, wt %:
Moisturizing component - 5 - 70,
Surface-active component - 0.5 - 3.0,
Flavor filler - 0.1 - 0.3,
Preservative - 0.01 - 0.5.

If therapeutic and prophylactic formulation were a paste, gel or liquid as moisturizing component there can be used one or several substances out of the group containing sorbitol, glycerine, polyethilenglycole, propylene glycol.

If therapeutic and prophylactic formulation were prepared in the form of a paste as abrasive component there can be used one or several substances selected out of the group including calcium carbonate, silica, aluminium oxide, aluminium hydroxide, polymethacrylate.

As gel-forming component of gel or paste there may be used one or several substances sorted out of the group comprising hydroxyethylcellulose, xanthum gum, guar gum, carboxymethylcellulose.

In the process of preparing of therapeutic and prophylactic formulation in the form of a paste, gel or liquid, there may be used surface-active components or their mixes such as sodium laurylsulfate, alkylamidobetaine, polysorbate-20, sodium lauryl sarcosinate.

In the process of preparing of therapeutic and prophylactic formulation in the form of a paste, gel or liquid formulations as a flavor filler there may be used one or several substances of the following group:
Essential oils such as, peppermint, spearmint, sage, eucalyptic, clove, skinleaf, anisic, tea tree, orange, grapefruit, lemon, bergamot oils; as well as menthol, carvone, anethol, eucalyptol, methyl salicylate;
Sweeteners - sodium saccharinate, potassium aspartame, stevioside, xylitol, potassium or sodium glycyrrhizate.

In the process of preparing of therapeutic and prophylactic formulation in the form of a paste, gel or liquid, function of preservatives may be performed by one or several substances selected out of the group consisting of methylparaben, propylparaben, or their sodium salts, and phenoxyethanol, benzole acid, sodium benzoate, potassium sorbate, triclosan.

### Realization of invention

Examples of therapeutic and prophylactic formulations in the form of a tooth-paste are illustrated in table 1.
Tooth-paste is prepared in the following way.
Weigh the required quantity of glycerine.
Add xanthum gum.
Mix to formation of homogeneous mass.

Weigh the required quantity of water in measuring bin and feed water into mixer.

Add sodium saccharate, parabens, sodium chloride, sorbitol, potassium metasilicate, sodium metasilicate into water.
Mix to formation of transparent solution.
Add xanthum gum suspension in glycerine to the solution obtained.
Mix till formation of homogeneous mass.
Degas and mix for 10 minutes till full deaeration of the mixture.

After add magnesium chloride (or magnesium glycerophosphate as like in example 4 or magnesium citrate as in example 5).
Mix for 15-20 minutes.
Then add silica (or calcium carbonate in examples 1 and 2).
Degas and mix for 30-40 minutes.
Homogenize tooth-paste with homogenizing pump for 10-20 minutes.
Add flavor and sodium laurylsulfate (or alkylamidobetaine in examples 4 and 5).
Mix till formation of homogeneous mass for 20-30 minutes.
The prepared tooth-paste is packed into tubes made of polymer material.

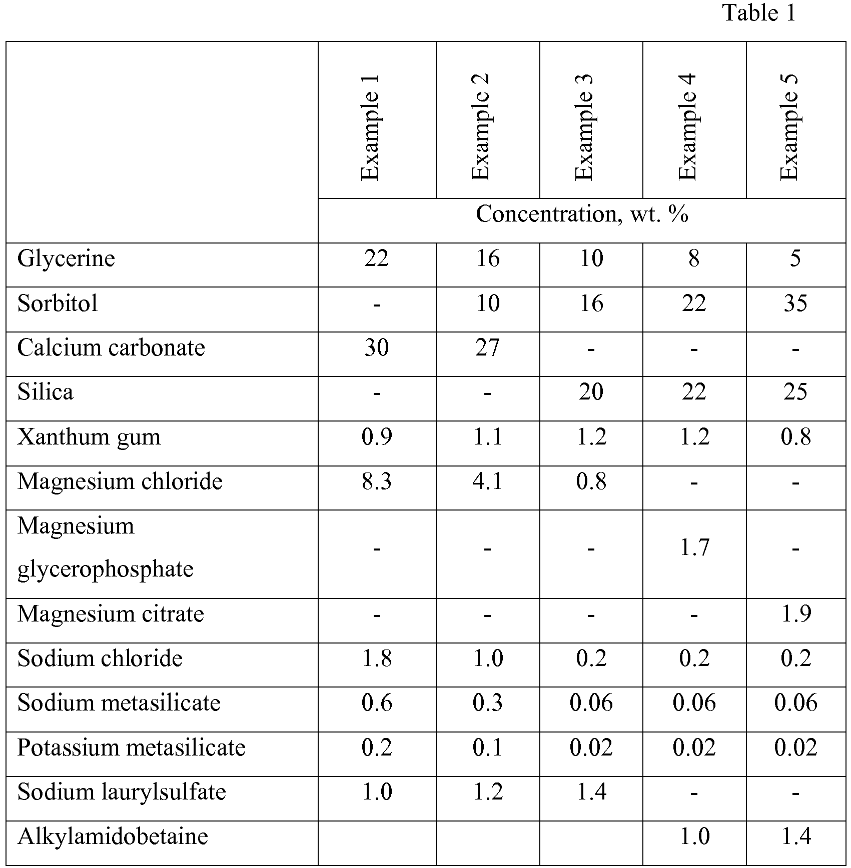

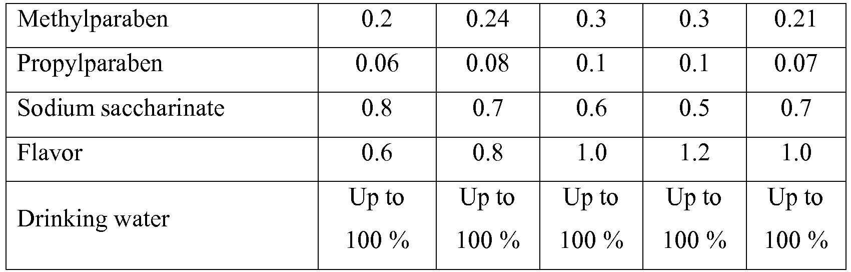

Examples of therapeutic and prophylactic formulations for mouth care in the form of tooth gel are shown in table 2.

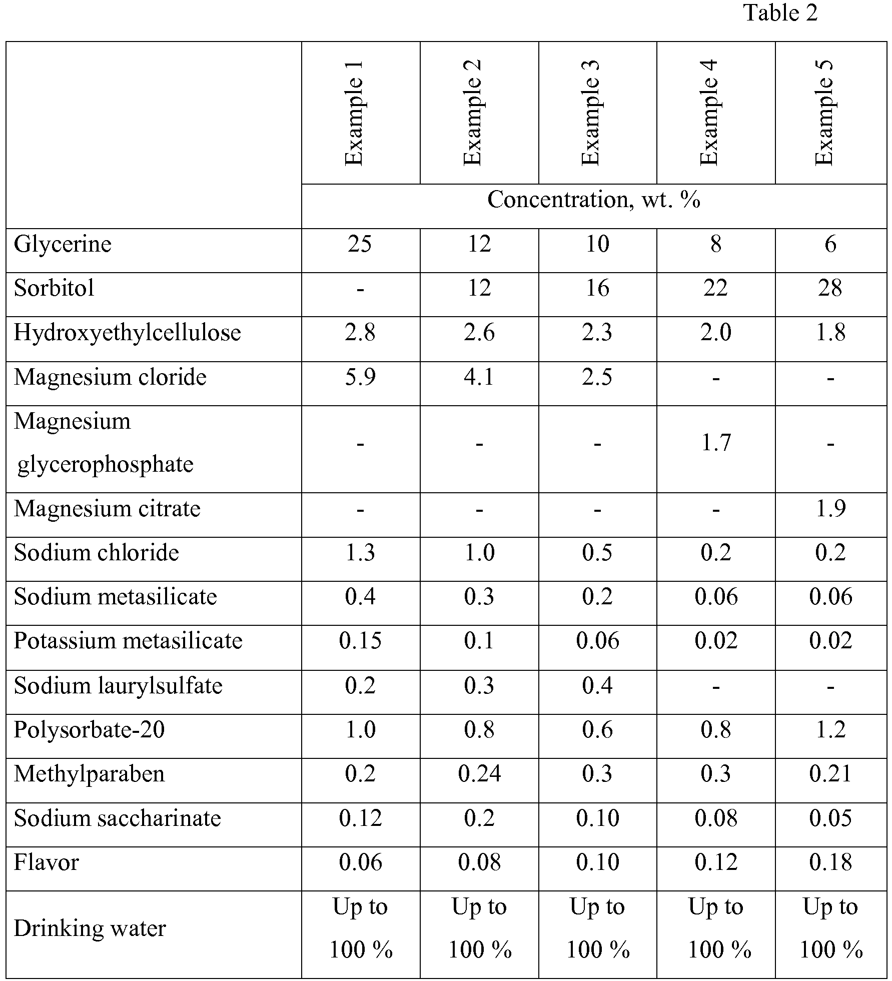

Formulation in the form of gel is prepared as follows.

Weigh the required quantity of water in measuring bin and feed it into mixer.

Add methylparaben, sorbitol, sodium saccharinate, sodium chloride, sodium metasilicate, potassium silicate into mixer.

Mix till formation of transparent solution for 20 minutes.

Prepare separately suspension of hydroxyethylcellulose in glycerine.

Add the suspension obtained into water solution.

Mix for 20-30 minutes till formation of homogeneous gel.

Add magnesium chloride (or magnesium glycerophosphate like in example 4 or magnesium citrate as in example 5) into the gel obtained.

Heat separately polysorbate-20 up to the temperature of 40-45 oC.

Add flavor and mix till formation of homogeneous mix for 10 minutes.

Add the obtained mix into gel and mix till formation of homogeneous mass for 20-30 minutes.

The prepared gel is packed into tubes made of polymer material.

Examples of therapeutic and prophylactic formulation for oral care in the form of liquid are listed in table 3.

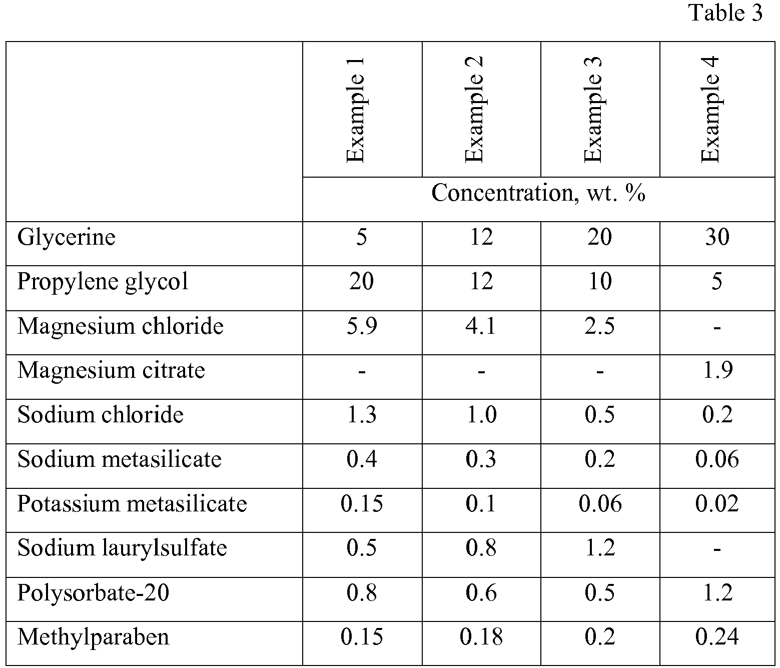

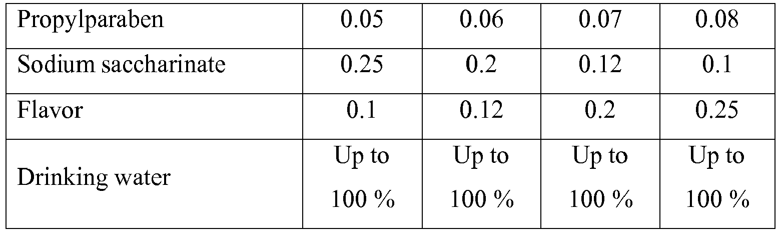

Formulation in the form of liquid is prepared in the following way.

Heat the required quantity of water in measuring bin to the temperature of 40-45 °C and feed into mixer.

Add glycerine, sodium chloride, sodium metasilicate, potassium metasilicate, sodium saccharinate.

Mix till formation of transparent solution for 20-30 minutes.

Dissolve separately parabens in propylene glycol.

Feed the obtained solution into the main mixer.

Mix till formation of transparent solution.

Cool the mix down to the temperature of 20-25 °C.

Add magnesium chloride (or magnesium citrate as in example 4).

Mix till formation of transparent or weakly opalescent solution.

Heat polysorbate-20 separately up to the temperature of 45-50 °C.

Add flavor.

Mix for 10 minutes till formation of homogeneous mass.

Add the mix obtained before.

Mix formulation for 20 minutes till formation of transparent or weakly opalescent solution.

Add sodium laurylsulfate, mix for 20 minutes and ladle into plastic bottles.

The effectiveness of therapeutic and prophylactic gels prepared in accordance with the proposed invention has been tested on group of volunteers in order to evaluate mineralizing influence.

There were used formulations of examples 1-3 from table 2. As a reference we use gel prepared in accordance with the analogue mentioned above (RU 2311168 C2, IPC 8 A61K 8/97, 2007). Formula of the gel is illustrated in table 4.

Clinical studies included 4 test-groups with 7 people aged from 18 to 21 (i.e. with teeth mineralization completed) in each group. All the participants had minimum 20 existing teeth with anatomic crowns. During the research values were double-estimated: before investigation and after investigation in three weeks. The test persons used gels. Method of application: gels were applied to preliminarily dried teeth with the help of individual mouthpiece tray for 15 minutes. On expiry of 15 minutes the trays were removed and teeth were refluxed with water.

Studies have been conducted by acid enamel biopsy method according to V.K.Leontjev and V.A.Distel' (1975), comprising application of strictly determined quantity of demineralizing liquid on the enamel, its sampling after a certain period of time and subsequent determination of calcium and phosphorus content in acid demineralisate. Quantitative analysis of elements in acidic biopsy material is performed by spectrophotometry.

**Table 4**

| Component | Concentration, wt.% |
|---|---|
| Glycerine | 10 |
| Sorbitol | 10 |
| Xylitol | 14 |
| Hydroxyethylcellulose | 2.2 |
| Magnesium chloride | 0.12 |
| Calcium glycerophosphate | 1.2 |
| Polysorbate-20 | 0.9 |
| Xanthum gum | 0.1 |
| Flavor | 0.4 |
| Drinking water | Up to 100 % |

Final average results of calcium and phosphorus content increase in teeth enamel of test-group participants (according to acid biopsy data), mol per liter are represented in table 5.

The investigations data testify to mineralizing ability of all pastes studied but to various extents.

Maximal calcium and phosphorus content increase can be noted in example 1. There were obtained worse results on gel applying in test-groups of examples 2 and 3. The least calcium and phosphorus increase was recorded in case of gel-analogue application. Thus, basing on the results of clinical studies it may be concluded that teeth enamel surface treatment by formulations containing sources of magnesium, sodium, silica and potassium in quantity 1-11 wt. % makes it possible to increase its mineralization degree.

Dental deposit cariogenicity determination has been carried out according to method suggested by J.L.Hardwick (1960). As a color indicator there was used methyl red. Enamel surface was treated by 1% glucose solution for 2 minutes with further dye application, 0.1 % methyl red water solution with time exposure of 1 minute. Reaction was evaluated as positive if dye color changed from yellow to red, that indicated to dental deposit pH and its cariogenic properties reduction. If there were no changes of color reaction was considered negative.

Macrohistochemical and bacteriological investigations of dental deposit on teeth enamel surface of lower jaw of patients applying gels as in examples 1-3, have shown absence of dental deposit cariogenicity and reduction in streptococci and stafilococci.

Thereby it has been elaborated high-efficiency and available therapeutic and prophylactic formulation for solid teeth tissues remineralization. Due to absence of fluorides it can be recommended as well to people suffering from fluorosis, endocrine system diseases for the purpose of caries prophylaxis and non-carious affections.

**Table 5**

| Gels | Before gel application | | In three weeks | |
|---|---|---|---|---|
| | Calcium | Phosphorus | Calcium | Phosphorus |
| Example 1 | 0.43 | 0.26 | 1.13 | 0.62 |
| | | | 2.63-fold increase of calcium | |
| | | | 2.38-fold increase of phosphorus | |
| Example 2 | 0.43 | 0.32 | 0.87 | 0.48 |
| | | | 2.02-fold increase of calcium | |
| | | | 1.5-fold increase of phosphorus | |
| Example 3 | 0.78 | 0.46 | 1.13 | 0.69 |
| | | | 1.45-fold increase of calcium | |
| | | | 1.5-fold increase of phosphorus | |
| Analogue | 0.58 | 0.37 | 0.67 | 0.42 |
| | | | 1.16-fold increase of calcium | |
| | | | 1.14-fold increase of phosphorus | |

## Claims

1. Therapeutic and prophylactic formulation for oral care which contains acceptable active and inert components, to the group of active components there belongs complex in quantity 1-11 wt. % including sources of elements of magnesium, sodium, silica and potassium with the ratios of atomic masses of elements, correspondingly, 21.3:9.3:1.67:1, herein as a source of magnesium element there is chosen magnesium chloride or magnesium glycerophosphate or magnesium citrate, as a source of sodium element there is chosen sodium chloride or sodium silicate, as a source of silica element there is chosen sodium metasilicate or potassium metasilicate, as a source of potassium element there is chosen potassium silicate.

2. Formulation according to point 1 **characterized by** containing a complex of active components in quantity of 2-9 wt. %

3. Formulation according to point 1 in the form of gel is **characterized by** containing following inert components, wt. %:
Moisturizing component - 5 - 70,
Gel-forming component - 0.5 - 3.0,
Surface-active component - 0.5-3.0,
Flavor filler - 0.05 - 0.3,
Preservative - 0.01 - 0.5.

4. Formulation according to point 1 in the form of paste is **characterized by** containing following inert components, wt %:
Abrasive component - 10 - 30,
Moisturizing component - 5 - 70,
Gel-forming component - 0.5 - 3.0,
Surface-active component - 0.5 - 3.0,
Flavor filler - 0.5 - 2.

5. Formulation according to point 1 in the form of liquid is **characterized by** containing following inert components, wt %:
Moisturizing component - 5 - 70,
Surface-active component - 0.5 - 3.0,
Flavor filler - 0.1 - 0.3,
Preservative - 0.01 - 0.5.

6. Formulation according to points 3 or 4 or 5 **characterized by** including as a moisturizing component one or several substances selected out of the group such as sorbitol, glycerine, polyethilenglycole, propylene glycol.

7. Formulation according to point 4 **characterized by** including as an abrasive component one or several substances selected out of the group such as calcium carbonate, silica, aluminium oxide, aluminium hydroxide, polymethacrylate.

8. Formulation according to points 3 or 4 **characterized by** including as a gel-forming component one or several substances selected out of the group such as hydroxyethylcellulose, xanthum gum, guar gum, carboxymethylcellulose.

9. Formulation according to points 3 or 4 or 5 **characterized by** including as a surface-active component one or several substances selected out of the group such as sodium laurylsulfate, alkylamidobetaine, polysorbate-20, sodium lauryl sarcosinate.

10. Formulation according to points 3 or 4 or 5 **characterized by** including as a flavor filler one or several substances selected out of the group comprising essential oils such as, peppermint, spearmint, sage, eucalyptic, clove, skinleaf, anisic, tea tree, orange, grapefruit, lemon, bergamot oils; as well as menthol, carvone, anethol, eucalyptol, methyl salicylate; sweeteners - sodium saccharinate, potassium aspartame, stevioside, xylitol, potassium or sodium glycyrrhizate.

11. Formulation according to points 3 or 4 or 5 **characterized by** including as preservatives one or several substances selected out of the group such as methylparaben, propylparaben, or their sodium salts, and phenoxyethanol, benzole acid, sodium benzoate, potassium sorbate, triclosan.

## Patentansprüche

1. Therapeutische und prophylaktische Formulierung zur Mundpflege, welche akzeptable aktive und inerte Komponenten enthält, zur Gruppe der aktiven Komponenten gehört ein Komplex in der Menge 1-11 wt. %, einschließlich Quellen an Elementen von Magnesium, Natrium, Silizium und Kalium in den Verhältnissen der Atommassenan der dementsprechenden Elemente, 21.3:9.3:1.67:1, welche hierin eine Quelle eines Magnesiumelements sind, wobei Magnesiumchlorid oder Magnesium-Glycerphosphat oder Magnesiumzitrat als Quelle für das Natriumelement gewählt wurde, als Quelle für ein Natriumelement wurde Natriumchlorid oder Natriumsilikat gewählt, als Quelle für ein Siliziumelement wurde ein Natriummetasilikat oder Potassiummetasilikat gewählt, als Quelle für das Kaliumelement wurde ein Kaliumsilikat gewählt.

2. Formulierung gemäß dem Punkt 1, **dadurch gekennzeichnet, dass** hier ein Komplex aktiver Komponenten in der Menge von 2-9 wt. % enthalten ist.

3. Formulierung gemäß Punkt 1, in Form eines Gels, **dadurch gekennzeichnet, dass** die folgenden inerten Komponenten enthalten sind; wt. %:
Befeuchtungskomponenten 5 - 70,
Gelformierende Komponente - 0,5 - 3,0,
Oberflächenaktive Komponente - 0,5 - 3,0, Aromafüllstoff - 0,05 - 0,3,
Konservierungsstoff - 0,01 - 0,5.

4. Formulierung gemäß Punkt 1 in Form einer Paste, **dadurch gekennzeichnet, dass** die folgenden inerten Komponenten enthalten sind, wt. %: Abrasivkomponente - 10 - 30,
Befeuchtungskomponente - 5 - 70,
Gelformierende Komponente - 0,5 - 3,0,
Oberflächenaktive Komponente - 0,5 - 3,0,
Aromafüllstoff - 0,5 - 2.

5. Formulierung gemäß Punkt 1 in Form eines Liquids, **dadurch gekennzeichnet, dass** die folgenden inerten Komponenten enthalten sind, wt. %:
Befeuchtungskomponente - 5 - 70,
Oberflächenaktive Komponente - 0,5 - 3,0,
Aromafüllstoff - 0,1 - 0,3,
Konservierungsstoff - 0,01 - 0,5.

6. Formulierung gemäß den Punkten 3 oder 4 oder 5, charakterisiert dadurch, dass einschließlich als eine Befeuchtigungskomponente eine oder mehrere Substanzen aus einer Gruppe, wie Sorbit, Glyzerin, Polyethilenglykol, Propylenglykol ausgewählt werden.

7. Formulierung gemäß Punkt 4, **dadurch gekennzeichnet, dass** eine Abrasivkomponente ein oder mehrere Substanzen inbegriffen sind, ausgewählt werden aus der Gruppe wie Calciumcarbonat, Silizium, Aluminiumoxid, Aluminiumhydroxid, Polymethacrylat.

8. Formulierung gemäß Punkten 3 oder 4, **dadurch gekennzeichnet, dass** als gelformierende Komponente eine oder mehrere Substanzen inbegriffen sind, die ausgewählt werden aus der Gruppe wie Hydroxyethylzellulose, Xanthangummi, Guargummi, Carboxymethylzellulose.

9. Formulierung gemäß Punkten 3 oder 4 oder 5, **dadurch gekennzeichnet, dass** als eine oberflächenaktive Komponente ein oder mehrere Substanzen inbegriffen sind, ausgewählt aus der Gruppe wie Natrium-Laurylsulfat, Alkylamidbetain, Polysorbat-20, Natriumlaurylsarcosinat.

10. Formulierung gemäß Punkten 3 oder 4 oder 5, **dadurch gekennzeichnet, dass** als ein Aromafüllstoff eine oder mehrere Substanzen inbegriffen sind, ausgewählt aus der Gruppe bestehend aus ätherischen Ölen, wie Pfefferminze, Grüne Minze, Salbei, Eukalyptus, Gewürznelke, Wintergrün, Anis, Teebaum, Orange, Grapefruit, Zitrone, Bergamotteöl; sowie Menthol, Carvone, Anethol, Eukalyptus, Methylsalisylat; Süßstoffe - Natriumsaccharinat, Kaliumaspartame, Steviosid, Xylit, Kalium- oder Natriumglycyrrhizat.

11. Formulierung gemäß Punkten 3 oder 4 oder 5, **dadurch gekennzeichnet, dass** als Konservierungsstoffe ein oder mehrere Substanzen inbegriffen werden, ausgewählt aus der Gruppe wie Methyparaben, Propylparaben oder deren Natriumsalze und Phenoxyethanol, Benzol, Säure, Natriumbenzoat, Kaliumsorbat, Triclosan.

## Revendications

1. Formulation thérapeutique et prophylactique pour les soins bucco-dentaires qui contient des composants actifs et inertes acceptables, au groupe de composants actifs appartient un complexe en une quantité de 1 à 11 % en poids comprenant des sources d'éléments de magnésium, de sodium, de silice et de potassium avec les rapports de masses atomiques des éléments correspondants 21.3:9.3:1.67:1, où est utilisé comme source d'élément de magnésium du chlorure de magnésium ou du glycérophosphate de magnésium ou du citrate de magnésium sélectionné, comme source d'élément de sodium du chlorure de sodium ou du silicate de sodium sélectionné, comme source d'élément de silice du métasilicate de sodium ou du métasilicate de potassium sélectionné, comme source d'élément de potassium du silicate de potassium sélectionné.

2. Formulation selon le point 1 **caractérisée en ce qu'**elle contient un complexe de composants actifs en une quantité de 2 à 9 % en poids.

3. Formulation selon le point 1 sous forme de gel est **caractérisée en ce qu'**elle contient des composants inertes suivants, en % en poids :
Composant hydratant : 5 à 70,
Composant gélifiant : 0,5 à 3,0,
Composant tensioactif : 0,5 à 3,0,
Liant aromatique : 0,05 à 0,3,
Conservateur : 0,01 à 0,5.

4. Formulation selon le point 1 sous forme de pâte est **caractérisée en ce qu'**elle contient des composants inertes suivants, en % en poids :
Composant abrasif : 10 à 30,
Composant hydratant : 5 à 70,
Composant gélifiant : 0,5 à 3,0,
Composant tensioactif : 0,5 à 3,0,
Liant aromatique : 0,5 à 2.

5. Formulation selon le point 1 sous forme de liquide est **caractérisée en ce qu'**elle contient des composants inertes suivants, en % en poids :
Composant hydratant : 5 à 70,
Composant tensioactif : 0,5 à 3,0,
Liant aromatique : 0,1 à 0,3,
Conservateur : 0,01 à 0,5.

6. Formulation selon les points 3 ou 4 ou 5 **caractérisée en ce qu'**elle comprend comme composant hydratant une ou plusieurs substances sélectionnées dans le groupe telle que le sorbitol, la glycérine, le polyéthylène glycol, le propylène glycol.

7. Formulation selon le point 4 **caractérisée en ce qu'**elle comprend comme composant abrasif une ou plusieurs substances sélectionnées dans le groupe telle que le carbonate de calcium, la silice, l'oxyde d'aluminium, l'hydroxyde d'aluminium, le polyméthacrylate.

8. Formulation selon les points 3 ou 4 **caractérisée en ce qu'**elle comprend comme composant gélifiant une ou plusieurs substances sélectionnées dans le groupe telle que l'hydroxyéthylcellulose, la gomme xanthane, la gomme de guar, la carboxyméthylcellulose.

9. Formulation selon les points 3 ou 4 ou 5 **caractérisée en ce qu'**elle comprend comme composant tensioactif une ou plusieurs substances sélectionnées dans le groupe telle que le laurylsulfate de sodium, des alkylamido bétaïnes, le polysorbate 20, le lauryl sarcosinate de sodium.

10. Formulation selon les points 3 ou 4 ou 5 **caractérisée en ce qu'**elle comprend comme liant aromatique une ou plusieurs substances sélectionnées dans le groupe comprenant des huiles essentielles telles que la menthe poivrée, la menthe verte, la sauge, l'eucalyptus, le clou de girofle, skinleaf, l'anis, l'arbre à thé, l'orange, le pamplemousse, le citron, les huiles de bergamote ; ainsi que le menthol, la carvone, l'anéthol, l'eucalyptol, le salicylate de méthyle ; édulcorants : saccharinate de sodium, acésulfame de potassium, aspartame, stévioside, xylitol, glycyrrhizate de sodium ou de potassium.

11. Formulation selon les points 3 ou 4 ou 5 **caractérisée en ce qu'**elle comprend comme conservateurs une ou plusieurs substances sélectionnées dans le groupe telle que le parahydroxybenzoate de méthyle, le parahydroxybenzoate de propyle, ou leurs sels de sodium, et le phénoxyéthanol, l'acide benzoïque, le benzoate de sodium, le sorbate de potassium, le triclosan.
